# EUROPEAN PATENT SPECIFICATION

(11) **EP 3 030 248 B1**
(45) Date of publication and mention of the grant of the patent: **24.06.2020**
(21) Application number: 14797471.1
(22) Date of filing: 09.05.2014
(51) Int. Cl.: A61K 36/13, A61K 31/19, A61K 31/20, A61P 31/04, A23L 33/115, A23D 9/00, A23K 10/32, C11C 1/04

(54) **TALL OIL FATTY ACID FOR USE IN TREATMENT AND ANIMAL FEED SUPPLEMENTS AND COMPOSITIONS THEREOF**
TALLÖLFETTSÄURE ZUR BEHANDLUNG UND IN TIERFUTTERZUSATZSTOFFEN SOWIE ZUSAMMENSETZUNGEN
ACIDE GRAS D' HUILE DE TALL POUR LE TRAITEMENT ET COMME ADDITIF DANS DES ALIMENTATIONS ANIMALES ET COMPOSITIONS

(30) Priority: 14.05.2013 FI 20135506
(43) Date of publication of application: 15.06.2016
(73) Proprietor: HANKKIJA OY, 05800 Hyvinkää (FI)
(72) Inventor: VUORENMAA, Juhani, FI-05800 Hyvinkää (FI); KETTUNEN, Hannele, FI-12400 Tervakoski (FI)
(74) Representative: Papula Oy
(86) International application number: PCT/FI2014/050346
(87) International publication number: WO 2014/184430

(56) References cited:
- EP-A2- 0 146 738
- WO-A1-02/02106
- WO-A1-2008/154522
- WO-A1-2008/154522
- WO-A1-2013/171370
- FI-B- 41 337
- US-A- 4 443 437
- US-A1- 2006 286 185
- US-A1- 2009 285 931
- Gudmundur Bergsson ET AL: "Antibacterial, Antiviral and Antifungal Activities of Lipids" In: "Lipids and Essential Oils as Antimicrobial Agents", 25 January 2011 (2011-01-25), John Wiley & Sons, Ltd, Chichester, UK, XP055323058, ISBN: 978-0-470-74178-8 pages 47-80, DOI: 10.1002/9780470976623.ch3, * page 48, paragraph 3 - page 51, paragraph 1 *
- DUNCAN D P: "TALL OIL FATTY ACIDS", NAVAL STORES, XX, XX, 1 January 1989 (1989-01-01), pages 346-349, XP002910189,
- 'Product Data Sheet SYLFAT® 2LTC tall oil fatty acid' 20 July 2009, XP008182610 Retrieved from the Internet: <URL:http://www.arizonachemical.com/Global/ PDS/EU_product_data_sheets/ SYLFAT%C2%AE%202LTC.pdf> [retrieved on 2014-09-01]
- E. VALKONEN ET AL: "Performance enhancing effect of a natural resin acid composition in broiler chickens under a variety of challenge conditions", PROCEEDINGS OF THE XVTH EUROPEAN POULTRY CONFERENCE, 17 September 2018 (2018-09-17), XP055658136, Dubrovnik, Croatia
- BRAUN H-S ET AL: "Dietary supplementation of essential oils in dairy cows: evidence for stimulatory effects on nutrient absorption", RED CLOVER POLYPHENOL OXIDASE AND LIPID METABOLISM, ANIMAL : AN INTERNATIONAL JOURNAL OF ANIMAL BIOSCIENCE FEB 2011, VOL. 5, NR. 4, PAGE(S) 512 - 521, 20 July 2018 (2018-07-20), pages 1-6, XP002788875, ISSN: 1751-732X, DOI: 10.1017/S1751731118001696

## Description

### FIELD OF THE INVENTION

The invention relates to a tall oil fatty acid comprising 1-10% resin acids for use in the prevention of growth of harmful bacteria in the animal digestive tract and/or in the prevention of intestinal disorders, and feed supplement and feed composition comprising said tall oil fatty acid.

### BACKGROUND OF THE INVENTION

Imbalances in microbial populations and growth of harmful bacteria in the digestive tract of animals can cause significant losses in animal growth and production. These imbalances manifest themselves as intestinal disorders such as diarrhea. While microbial infections of animals have been prevented by the use of e.g. antibiotics and other agents that prevent the growth of microorganisms, stricter regulations on their use are expected. Generally, there is an increasing demand for ingredients for use in animal feeding that can modulate the microbial population in the animal digestive tract but which are readily available, well tolerated and environmentally friendly.

Fractional distillation of crude tall oil, obtained as a by-product of the Kraft process of wood pulp manufacture, produces distilled tall oil (DTO) which typically comprises over 10% resin acids and less than 90% fatty acids. Further refinement of distilled tall oil produces tall oil fatty acid (TOFA), which is available in a variety of compositions differing in the fatty acids and resin acids content. Because TOFA is an inexpensive source of fatty acids, it has previously been used in animal nutrition as an energy source. For instance, GB 955316 discloses the use of alkali metal salts of tall oil fatty acids to improve weight gain and nitrogen retention in ruminant animals.

Document US 2009/285931 A1 discloses a method for enhancing feed efficiency and reducing enteric methane production in livestock, comprising a formulation of natural plants such as *Camelina sativa* containing fatty acids especially C-18 fatty acids (particularly alpha-linolenic) and plant extracts and chemicals, including propionatic acid glycerol, that when feed to ruminants results in decreased enteric methane production and improved feed efficiency.

### PURPOSE OF THE INVENTION

The purpose of the invention is to provide a new type of tall oil fatty acid/feed supplement for use in the prevention of growth of harmful bacteria in the animal digestive tract and/or in the prevention of intestinal disorders.

The present inventors have surprisingly found that TOFA prevents the growth of harmful bacteria in the animal digestive tract and/or prevents intestinal disorders.

### SUMMARY

The tall oil fatty acid according to the present invention is characterized by what is presented in claim 1.

The feed supplement according to the present invention is characterized by what is presented in claim 7.

The feed composition according to the present invention is characterized by what is presented in claim 12.

### DETAILED DESCRIPTION OF THE INVENTION

FIG 1a The turbidity change during 8 hours of Cl. perfringens growth as a response to TOFA and digested TOFA concentrations.
FIG 1b Gas production during 8 hours by *Cl. perfringens* growth as a response to TOFA and digested TOFA concentrations.
FIG 2a The turbidity change during 8 hours of Cl. perfringens growth as a response to TOFA and test products at dose 1.
FIG 2b The turbidity change during 8 hours of Cl. perfringens growth as a response to TOFA and test products at dose 2.
FIG 2c The turbidity change during 8 hours of *S. aureus* as a response to TOFA and test products at dose 1.
FIG 2d The turbidity change during 8 hours of *S. aureus* as a response to TOFA and test products at dose 2.
FIG 2e The turbidity change during 8 hours of *S. suis* as a response to TOFA and test products at dose 1.
FIG 2f The turbidity change during 8 hours of *C S. suis* as a response to TOFA and test products at dose 2.

The present invention is based on the realization that tall oil fatty acid comprising 1-10% (w/w) of resin acids can be used in the prevention of growth of harmful bacteria in the animal digestive tract and/or in the prevention of intestinal disorders.

The term "tall oil fatty acid" or "TOFA" should be understood as referring to a composition obtained by distillation of crude tall oil and further refinement of distilled tall oil. TOFA typically comprises 90-98% (w/w) fatty acids. Further, TOFA may comprise 1-10% (w/w) resin acids.

Tthe tall oil fatty acid for use according to the present invention comprises 1-10% (w/w) of resin acids.

In one embodiment of the present invention, TOFA comprises 2-9 % (w/w) resin acids.

In one embodiment of the present invention, TOFA comprises 5-9% (w/w) resin acids.

In this context, the term "resin acids" should be understood as referring to a complex mixture of various acidic compounds comprised by tall oil which share the same basic skeleton including a three-fused ring. The exact composition of the resin acids present in TOFA varies e.g. according to the species of the trees the TOFA is obtained from and the processing conditions under which it is manufactured. Resin acids typically include compounds such as abietic acid, dehydroabietic acid, levopimaric acid, neoabietic acid, pimaric acid and isopimaric acid, only to mention a few.

In one embodiment of the present invention, TOFA comprises 90-98% (w/w) of fatty acids.

The tall oil fatty acid (TOFA) is produced by refinement from distilled tall oil. Distilled tall oil (DTO) is produced by fractional distillation from crude tall oil, obtained as a by-product of the Kraft process of wood pulp manufacture.

In one embodiment of the present invention, the TOFA, for use according to the present invention is dried. The TOFA can be dried by spray drying, drum drying or by any other known suitable drying method.

The present invention also relates to a feed supplement comprising the tall oil fatty acid comprising 1-10% (w/w) resin acids according to the invention.

The feed supplement is effective in the prevention of growth of harmful bacteria, for prevention of intestinal disorders, in the modulation of microbial population of the animal digestive tract, in enhancing rumen fermentation and/or lowering rumen methane production.

The feed supplement comprises a tall oil fatty acid which comprises 1-10% (w/w) resin acids.

In one embodiment of the present invention, the feed supplement comprises a tall oil fatty acid which comprises 2-9 % (w/w) resin acids.

In one embodiment of the present invention, the feed supplement comprises a tall oil fatty acid which comprises 5-9% (w/w) resin acids.

In this context, the term "feed supplement" should be understood as referring to a composition that may be added to a feed or used as such in the feeding of animals. The feed supplement may comprise different active ingredients. The feed supplement may be added in the feed in a concentration of 0.0001 - 5 kg//ton of dry weight, preferably 0.005 - 1 kg/ton of the dry weight of the total amount of the feed. The TOFA or the feed supplement comprising the TOFA according to the invention may be added to the feed or feed supplement as such, or it may in general be further processed as desired.

Further, the TOFA or the feed supplement comprising the TOFA according to the invention may be added to the feed or feed supplement, or it may be administered to an animal separately (i.e. not as a part of any feed composition).

In this context, the term "feed composition" or "feed" should be understood as referring to the total feed composition of an animal diet or to a part thereof, including e.g. supplemental feed, premixes and other feed compositions. The feed may comprise different active ingredients.

In one embodiment of the present invention, the feed supplement comprises TOFA which is absorbed into a carrier material suitable for the feed composition such as sugarbeet pulp.

In one embodiment of the present invention, the feed supplement comprises TOFA which is dried.

The present invention also relates to a feed composition comprising the feed supplement comprising the tall oil fatty acid comprising 1-10% (w/w) resin acids according to the invention.

In one embodiment of the present invention, the feed composition comprises the feed supplement in an amount of 0.00001 - 0.5 % (w/w), of the dry weight of the total amount of the feed.

In one embodiment of the present invention, the feed composition comprises the feed supplement in an amount of 0.0005 - 0.1 % (w/w) of the dry weight of the total amount of the feed.

In one embodiment of the present invention, the method of producing a tall oil fatty acid or feed supplement further comprises a step of drying. The dying can be carried out by spray drying, drum drying or by any other known drying method.

A method of preventing the growth of harmful bacteria in the animal digestive tract and/or preventing intestinal disorders, comprising the step of administering to an animal the tall oil fatty acid is disclosed herein.

In this context, the term "harmful bacteria" should be understood as referring to any bacteria that is capable of affecting the digestive tract or health of an animal in an adverse manner, including competition for nutrients with the host animal. In this context, the term "microbial population" should be understood as referring to the microorganisms that inhabit the digestive tract, including the Bacteria and Archaea domains and microscopic members of the Eukaryote domain and also intestinal parasites. The microbial population will vary for different animal species depending on e.g. the health of an animal and on environmental factors.

In this context, the term "intestinal disorder" should be understood as referring to various disorders of the digestive tract in an animal, including e.g. diarrhea and other intestinal health problems.

In this context, the term "animal" should be understood as referring to all kinds of different animals, such as monogastric animals, ruminants, fur animals, pets and aquaculture. Non-limiting examples of different animals, including offspring, are cows, beef cattle, pigs, poultry, sheep, goats, horses, foxes, dogs, cats and fish.

In one embodiment of the present invention, the TOFA is administered to an animal in an effective amount. In a further embodiment, the TOFA is administered in a therapeutically effective amount.

The present invention has a number of advantages. TOFA is a readily available, natural, low-cost and environmentally friendly material. Further, it is non-toxic and well tolerated. Subsequently, other benefits of the invention are e.g. improved animal health and productivity, higher product quality, uniformity, food and product safety. The invention also allows the production of feed compositions and supplements at low cost.

The embodiments of the invention described hereinbefore may be used in any combination with each other. Several of the embodiments may be combined together to form a further embodiment of the invention. A product, a method or a use, to which the invention is related, may comprise at least one of the embodiments of the invention described hereinbefore.

### EXAMPLES

In the following, the present invention will be described in more detail.

### EXAMPLE 1.

### Pathogen inhibition test

*Clostridium perfringens* is a pathogenic bacterium that causes necrotic enteritis in broiler chicks and other species of poultry. This experiment was conducted to study the inhibition of *Cl. perfringens* by TOFA with 5 % resin acids.

The efficiency of untreated and digested test compounds was tested in a *Cl. perfringens* growth inhibition test that measures both the turbidity of the clostridial culture medium as a result of increased number of bacterial cells in a unit volume of medium, and the cumulative gas production during the simulation.

There were four treatments in the test: control, control/ethanol, TOFA 5 % and pre-digested TOFA 5% and the TOFA products were tested in two concentrations. To make the untreated TOFA 5% soluble in the water phase of the simulation medium, it was first diluted with ethanol. The digested TOFA product was diluted in sterile water.

*Gastrointestinal digestion of the TOFA: The* tall oil was digested in 5% and 1% stock solutions, starting with pepsin-HCl digestion (pH 2.5) at +37°C treatment for 3 hours, followed by neutralization of the digesta with NaOH (pH 6.5) and the treatment with bile acids and pancreatin for additional 3 hours at +37°C. This digestion mimics the gastric and small-intestinal digestion of monogastric animals. Digestion was made to evaluate whether the product would resist the conditions of upper gastrointestinal tract before entering to the distal intestine with higher microbial activity.

The simulation was conducted in 25-ml glass bottles containing 15 ml of sterile anaerobic TSGY-media (tryptic soy broth -yeast extract media with glucose) and bottles were enclosed with air-tight stoppers to ensure anaerobic conditions throughout the experiment. At the beginning of simulation 0.1 % inoculums of the overnight grown *Cl. perfringens* culture was injected to TSGY-bottles. Test compounds or ethanol was added in 150 µl final volume from the respective stock solution according to the treatment. The simulation bottles were randomized to avoid artificial bias between treatments. The bottles were kept at even temperature of 37 °C and mixed 1 min before turbidity measurement at each time point. The total simulation time was 8h.

The turbidity was measured at the time points of 0.5, 3, 6 and 8 hours. The turbidity (optical density, OD) of growth media increases proportionally as *Cl. perfringens* cell number and cell density increases. Sometimes the highest concentrations of test compounds affect to the turbidity already in the beginning of simulation regardless of bacterial growth, and therefore the turbidity change of each separate simulation bottle is more informative in comparing the different test compounds or doses. Total gas production was measured at the end of the 8h simulation as an indicator of growth efficiency, since *Cl. perfringens* produces detectable amounts of gas due to the active metabolism during exponential growth.

### Results

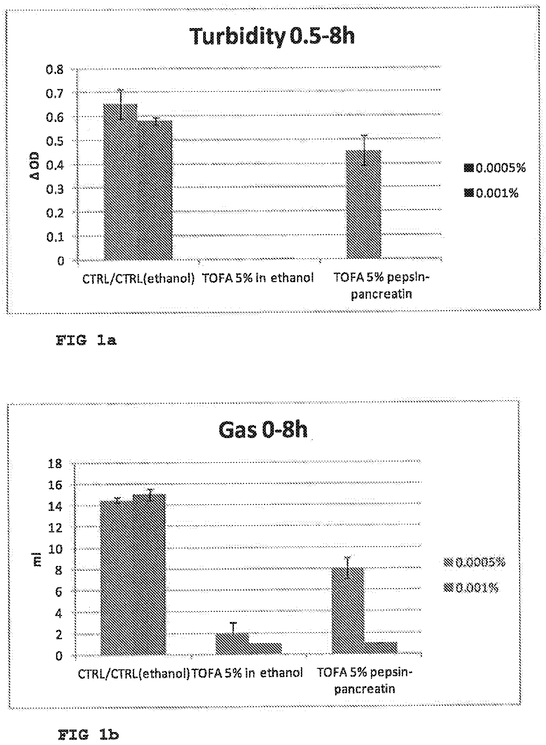

The results are illustrated in Figure 1a and 1b. Both untreated and digested TOFA treatments effectively inhibited the growth of *Cl. perfringens* almost completely still in the concentration of 0.001%, which was detected as lack of turbidity change in 8 hours (Figure 1a) and the production of less than 2 ml gas (Figure 1b).

The concentration 0.05% of TOFA, which is not shown in the figures, totally prevented the growth of *Cl. perfringens.*

The results show that TOFA resists gastrointestinal digestion and maintains its efficacy against the growth of *Cl. perfringens.* This result shows that the TOFA prevents or alleviates the onset of necrotic enteritis if given to broiler chicks or other species of poultry in the feed.

The experiment shows that the TOFA is very effective against the growth of *Cl. perfringens,* and that most of its activity can resist gastrointestinal digestion.

### EXAMPLE 2.

### Pathogen inhibition test

This experiment was conducted to compare the efficacy of TOFA with 8.5 % resin acids and competing products for their ability to inhibit the growth of pure cultures of three Gram-positive pathogens: *Clostridium perfringens, Staphylococcus aureus and Streptococcus suis in vitro.* The competing products were commercial natural plant extracts and a medium-chain fatty acid product. Plant extracts A-C are merchandised to have inhibitory effects against Gram+ pathogenic bacteria and they can be used e.g. in management of coccidiosis risk. Before the simulation, the bacteria were grown overnight as pure cultures in their specific growth medium. The bacterial cultures were used as inoculant in the experiment.

Products and product doses are presented in Table 1.

| Product | Dose 1 (kg/ton of feed) | Dose 2 (kg/ton of feed) |
|---|---|---|
| TOFA 8.5 % resin acids | 0.25 | 0.50 |
| Plant (Oregano) extract A | 0.25 | 0.50 |
| Plant extract B | 0.25 | 0.50 |
| Plant extract C | 0.50 | 1.00 |
| Medium chain fatty acid product (MCFA) | 0.25 | 0.50 |

The test products were first weighed into the glass bottles. 15 ml of bacterial growth medium was added. Bottles for *Cl. perfringens* and *S. aureus* were prepared in anaerobic glove box, while the bottles for *S. suis* and *B. cereus* were prepared in aerobic environment. Next, the bottles were enclosed with air-tight stoppers to ensure anaerobic conditions throughout the experiment for *Cl. perfringens* and *S. aureus.* A needle was pushed through the stoppers of the two aerobic bacteria to ensure oxygen supply for the culture. 150 ml of bacterial culture (see above) was added into each bottle to act as an inoculum (1% of the volume). The simulation time was calculated starting from the time of inoculating each vessel. During the simulation, the bottles were kept at 37°C temperature in a constant, slow shaking for eight hours. Optical density was measured at the time points of 0, 2, 4, 6 and 8 hours. The turbidity (optical density, OD) of growth media increases proportionally as bacterial cell density increases.

Each product was tested at two concentrations and three replicates per concentration. Dose 2 was the recommended dose of the commercial products. Each product concentration had also a control vessel into which microbes were not included (one replicate/product/dose). These treatments controlled for any potential increase in the cloudiness that the test products may have induced into the growth medium during the simulation time. The total number of simulation vessels was 123 per bacterial species.

### Results

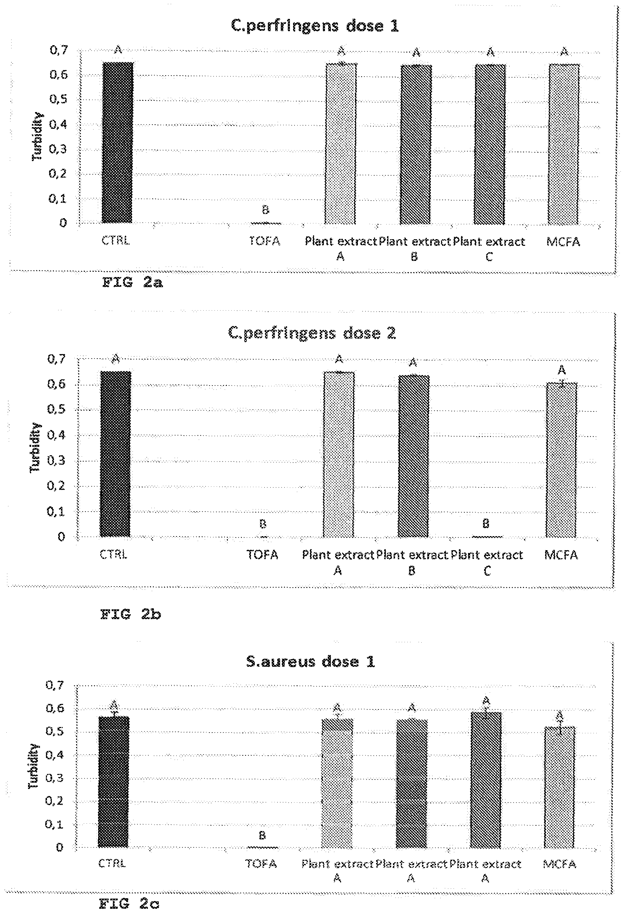

The results are illustrated in Figures 2a to 2f. The TOFA of the invention totally inhibited the growth of *Cl. perfringens* at both product levels at the 8-hour time point (Figure 2a and 2b).

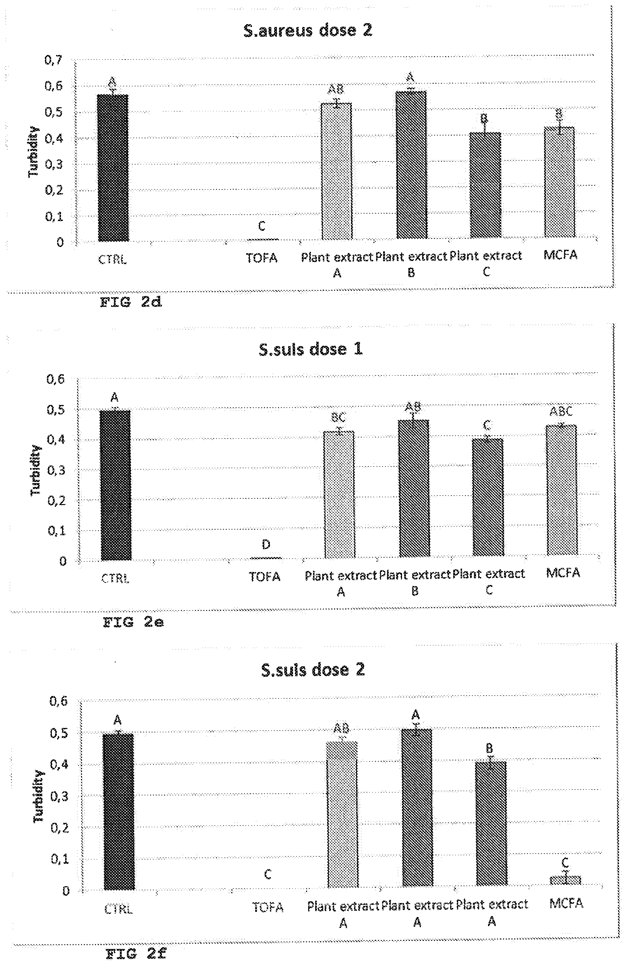

*S. aureus* was not able to grow at all in the presence of TOFA at the studied concentrations, while the other products showed no inhibition at dose 1 (Figure 2c). Two of the other products showed partial inhibition at product dose 2 (Figure 2d).

TOFA fully prevented the growth of *Streptococcus suis* during the 8-hour simulation at both product doses (Figure 2e and 2f). At dose 2, MCFA product efficiently inhibited the growth of *S. suis* at the 8-hour time point (Figure 2f).

The experiment shows that the TOFA is much more effective against the growth of *Cl. perfringens, Staphylococcus aureus and Streptococcus suis* as the commercial plant extracts A-C claiming inhibitory effects against Gram+ pathogenic bacteria.

It is obvious to a person skilled in the art that, with the advancement of technology, the basic idea of the invention may be implemented in various ways. The invention and its embodiments are thus not limited to the examples described above; instead they may vary within the scope of the claims.

## Claims

1. A tall oil fatty acid comprising 1-10% (w/w) resin acids for use in the prevention of growth of harmful bacteria in the animal digestive tract and/or in the prevention of intestinal disorders.

2. The tall oil fatty acid for use according to claim 1 in the prevention of growth of harmful bacteria in the animal digestive tract.

3. The tall oil fatty acid for use according to claim 1 or 2, **characterized in that** it comprises 2-9 % (w/w) resin acids.

4. The tall oil fatty acid for use according to any of preceding claims 1 - 3, **characterized in that** it comprises 5-9% (w/w) resin acids.

5. The tall oil fatty acid for use according to any of preceding claims 1 - 4, **characterized in that** it comprises 90-98% (w/w) fatty acids.

6. The tall oil fatty acid for use according to any of preceding claims 1 - 5, **characterized in that** it is dried.

7. A feed supplement comprising a tall oil fatty acid comprising 1-10% (w/w) resin acids for use in the prevention of growth of harmful bacteria in the animal digestive tract and/or in the prevention of intestinal disorders.

8. The feed supplement for use according to claim 7 in the prevention of growth of harmful bacteria in the animal digestive tract.

9. The feed supplement for use according to claim 7 or 8, **characterized in that** the tall oil fatty acid comprises 2-9 % (w/w)or 5-9% (w/w) resin acids.

10. The feed supplement for use according to any of preceding claims 7 - 9, **characterized in that** the tall oil fatty acid is dried.

11. The feed supplement for use according to any of preceding claims 7 - 10, **characterized in that** the tall oil fatty acid is absorbed into a carrier material.

12. A feed composition comprising a feed supplement comprising a tall oil fatty acid comprising 1-10% (w/w) resin acids for use in the prevention of growth of harmful bacteria in the animal digestive tract and/or in the prevention of intestinal disorders.

13. A feed composition for use according to claim 12 in the prevention of growth of harmful bacteria in the animal digestive tract.

14. A feed composition for use according to claim 12 or 13, **characterized in that** it comprises a feed supplement in an amount of 0.00001 - 0.5 % (w/w) of the dry weight of the total amount of feed.

15. A feed composition for use according to any of claims 12 - 14, **characterized in that** it comprises a feed supplement in an amount of 0.0005 - 0.1 % (w/w) of the dry weight of the total amount of feed.

## Patentansprüche

1. Tallölfettsäure, umfassend 1-10 Gew.-% Harzsäuren zur Verwendung bei der Verhinderung des Wachstums von schädlichen Bakterien im Verdauungstrakt von Tieren und/oder bei der Verhinderung von Darmstörungen.

2. Tallölfettsäure zur Verwendung nach Anspruch 1 bei der Verhinderung des Wachstums von schädlichen Bakterien im Verdauungstrakt von Tieren.

3. Tallölfettsäure zur Verwendung nach Anspruch 1 oder 2, **dadurch gekennzeichnet, dass** sie 2-9 Gew.-% Harzsäuren umfasst.

4. Tallölfettsäure zur Verwendung nach einem der vorhergehenden Ansprüche 1 - 3, **dadurch gekennzeichnet, dass** sie 5-9 Gew.-% Harzsäuren umfasst.

5. Tallölfettsäure zur Verwendung nach einem der vorhergehenden Ansprüche 1 - 4, **dadurch gekennzeichnet, dass** sie 90-98 Gew.-% Fettsäuren umfasst.

6. Tallölfettsäure zur Verwendung nach einem der vorhergehenden Ansprüche 1 - 5, **dadurch gekennzeichnet, dass** sie getrocknet ist.

7. Futterzusatz, umfassend eine Tallölfettsäure, umfassend 1-10 Gew.-% Harzsäuren zur Verwendung bei der Verhinderung des Wachstums von schädlichen Bakterien im Verdauungstrakt von Tieren und/oder bei der Verhinderung von Darmstörungen.

8. Futterzusatz zur Verwendung nach Anspruch 7 bei der Verhinderung des Wachstums von schädlichen Bakterien im Verdauungstrakt von Tieren.

9. Futterzusatz zur Verwendung nach Anspruch 7 oder 8, **dadurch gekennzeichnet, dass** die Tallölfettsäure 2-9 Gew.-% oder 5-9 Gew.-% Harzsäuren umfasst.

10. Futterzusatz zur Verwendung nach einem der vorhergehenden Ansprüche 7 - 9, **dadurch gekennzeichnet, dass** die Tallölfettsäure getrocknet ist.

11. Futterzusatz zur Verwendung nach einem der vorhergehenden Ansprüche 7 - 10, **dadurch gekennzeichnet, dass** die Tallölfettsäure in ein Trägermaterial absorbiert ist.

12. Futterzusammensetzung, umfassend einen Futterzusatz, umfassend eine Tallölfettsäure, umfassend 1-10 Gew.-% Harzsäuren zur Verwendung bei der Verhinderung des Wachstums von schädlichen Bakterien im Verdauungstrakt von Tieren und/oder bei der Verhinderung von Darmstörungen.

13. Futterzusammensetzung zur Verwendung nach Anspruch 12 bei der Verhinderung des Wachstums von schädlichen Bakterien im Verdauungstrakt von Tieren.

14. Futterzusammensetzung zur Verwendung nach Anspruch 12 oder 13, **dadurch gekennzeichnet, dass** sie einen Futterzusatz in einer Menge von 0,00001 - 0,5 Gew.-% des Trockengewichts der Gesamtmenge von Futter umfasst.

15. Futterzusammensetzung zur Verwendung nach einem der Ansprüche 12 oder 14, **dadurch gekennzeichnet, dass** sie einen Futterzusatz in einer Menge von 0,0005 - 0,1 Gew.-% des Trockengewichts der Gesamtmenge von Futter umfasst.

## Revendications

1. Acide gras de tallöl comprenant 1 à 10 % (p/p) d'acides résiniques pour une utilisation dans la prévention de la croissance de bactéries nocives dans le tube digestif animal et/ou dans la prévention de troubles intestinaux.

2. Acide gras de tallöl pour une utilisation selon la revendication 1 dans la prévention de la croissance de bactéries nocives dans le tube digestif animal.

3. Acide gras de tallöl pour une utilisation selon la revendication 1 ou 2, **caractérisé en ce qu'**il comprend 2 à 9 % (p/p) d'acides résiniques.

4. Acide gras de tallöl pour une utilisation selon l'une quelconque des revendications 1 à 3 précédentes, **caractérisé en ce qu'**il comprend 5 à 9 % (p/p) d'acides résiniques.

5. Acide gras de tallöl pour une utilisation selon l'une quelconque des revendications 1 à 4 précédentes, **caractérisé en ce qu'**il comprend 90 à 98 % (p/p) d'acides gras.

6. Acide gras de tallöl pour une utilisation selon l'une quelconque des revendications 1 à 5 précédentes, **caractérisé en ce qu'**il est séché.

7. Complément alimentaire comprenant un acide gras de tallöl comprenant 1 à 10 % (p/p) d'acides résiniques pour une utilisation dans la prévention de la croissance de bactéries nocives dans le tube digestif animal et/ou dans la prévention de troubles intestinaux.

8. Complément alimentaire pour une utilisation selon la revendication 7 dans la prévention de la croissance de bactéries nocives dans le tube digestif animal.

9. Complément alimentaire pour une utilisation selon la revendication 7 ou 8, **caractérisé en ce que** l'acide gras de tallöl comprend 2 à 9 % (p/p) ou 5 à 9 % (p/p) d'acides résiniques.

10. Complément alimentaire pour une utilisation selon l'une quelconque des revendications 7 à 9 précédentes, **caractérisé en ce que** l'acide gras de tallöl est séché.

11. Complément alimentaire pour une utilisation selon l'une quelconque des revendications 7 à 10 précédentes, **caractérisé en ce que** l'acide gras de tallöl est absorbé dans un matériau support.

12. Composition alimentaire comprenant un complément alimentaire comprenant un acide gras de tallöl comprenant 1 à 10 % (p/p) d'acides résiniques pour une utilisation dans la prévention de la croissance de bactéries nocives dans le tube digestif animal et/ou dans la prévention de troubles intestinaux.

13. Composition alimentaire pour une utilisation selon la revendication 12 dans la prévention de la croissance de bactéries nocives dans le tube digestif animal.

14. Composition alimentaire pour une utilisation selon la revendication 12 ou 13, **caractérisée en ce qu'**elle comprend un complément alimentaire en une quantité de 0,00001 à 0,5 % (p/p) du poids sec de la quantité totale d'aliment.

15. Composition alimentaire pour une utilisation selon l'une quelconque des revendications 12 à 14, **caractérisée en ce qu'**elle comprend un complément alimentaire en une quantité de 0,0005 à 0,1 % (p/p) du poids sec de la quantité totale d'aliment.
